# EUROPÄISCHE PATENTANMELDUNG

(11) **EP 2 199 268 A1**
(43) Veröffentlichungstag der Anmeldung: **23.06.2010**
(21) Anmeldenummer: 08021782.1
(22) Anmeldetag: 16.12.2008
(51) Int. Cl.: C07C 1/20, C07C 9/16, C07C 9/22

(54) **Verzweigte Kohlenwasserstoffe und deren Verwendung**

(71) Anmelder: Cognis IP Management GmbH, 40589 Düsseldorf (DE)
(72) Erfinder: Dierker, Markus, 40593 Düsseldorf (DE); Samorski, Markus, 40217 Düsseldorf (DE)

(57) **Zusammenfassung**

Die vorliegende Erfindung betrifft verzweigte Kohlenwasserstoffe, erhältlich durch Dehydroxymethylierung von Edukten ausgewählt aus der Gruppe bestehend aus
a) **verzweigten Alkoholen** der allgemeinen Formel R₁-OH, wobei R₁ für einen verzweigten Alkyl und/oder Alkenylrest mit 10 bis 29 C Atomen steht,
b) **verzweigten primären Diolen** der allgemeinen Formel HO-X-OH, wobei X für einen verzweigten Alkyl und/oder Alkenylrest mit 11 bis 44 C Atomen steht
c) **verzweigten primären Triolen** mit 12 bis 66 C Atomen

sowie deren Verwendung in kosmetischen und/oder pharmazeutischen Zubereitungen.

## Beschreibung

Die vorliegende Erfindung betrifft verzweigte Kohlenwasserstoffe, ihre Verwendung in kosmetischen und/oder pharmazeutischen Zubereitungen, sowie kosmetische und/oder pharmazeutische Zubereitungen, enthaltend verzweigte Kohlenwasserstoffe.

### Stand der Technik

Sensorisch leichte Ölkörper, so genannte "light emollients", werden von der kosmetischen Industrie in einer Vielzahl von Formulierungen verwendet. Insbesondere für die dekorative Kosmetik bzw. in pflegenden Formulierungen werden so genannte "leichte" Komponenten eingesetzt. Bei diesen Komponenten kann es sich beispielsweise um flüchtige, cyclische Silikone (z.B. Cyclopentasiloxan oder Cyclomethicone) oder Kohlenwasserstoffe aus petrochemischen Prozessen handeln. Bei den zuletzt genannten Stoffen handelt es sich aufgrund ihrer Herstellung überwiegend um Gemische aus linearen, cyclischen und verzweigten Kohlenwasserstoffen, deren Flammpunkt durchaus unter 50 °C (wie z.B. beim Isododecan) liegen kann. Beispiele und anwendungstechnische Beschreibungen derartiger Formulierungen können in Standardwerken, wie zum Beispiel: **,**Handbook of Cosmetic Science and Technology', A. Barel, M. Paye, H. Maibach, Marcel Dekker Inc. 2001 nachgelesen werden. Aus toxikologischen, ökologischen bzw. sicherheitstechnischen Gründen besteht jedoch in Zukunft Bedarf nach alternativen Rohstoffen für derartige Formulierungsaufgaben.

In kosmetischen und pharmazeutischen Zubereitungen werden unter der Bezeichnung "Mineral Öl" die aus mineralischen Rohstoffen (Erdöl, Braun- u. Steinkohlen) gewonnenen flüssigen Destillations-Produkte, die im Wesentlichen aus Gemischen von gesättigten Kohlenwasserstoffen mit linearer, cyclischer und/oder verzweigter Struktur bestehen, eingesetzt. Diese Kohlenwasserstoff Gemische müssen jedoch aufwendig gereinigt und chemisch modifiziert werden, bevor sie den Anforderungen an kosmetische Rohstoffe entsprechen.

Die Aufgabe der Erfindung bestand darin, alternative Rohstoffe zu finden, die ökologisch bzw. toxikologisch unbedenklich sind. Dabei war es insbesondere von Interesse Rohstoffe bereit zu stellen, welche ohne aufwendige Reinigungsschritte direkt in kosmetischen bzw. pharmazeutischen Zubereitungen eingesetzt werden können. Vorzugsweise sollten diese Rohstoffe auf Basis nachwachsender Rohstoffe erhältlich sein. Diese Rohstoffe sollten in typischen kosmetischen und/oder pharmazeutischen Formulierungen ohne anwendungstechnische Einschränkungen direkt eingesetzt werden können. Darüber hinaus sollten die Rohstoffe gegenüber den Kohlenwasserstoff Gemischen des Standes der Technik eine verbesserte Sensorik aufweisen, wünschenswert war auch, dass diese Rohstoffe eine bessere Hautverträglichheit aufweisen. Von besonderem Interesse war es, Rohstoffe bereit zu stellen, welche hinsichtlich ihrer formulierungstechnischen oder sensorischen Einsatzmöglichkeiten mit Silikonölen, insbesondere mit niedrigviskosen Silikonölen, wie z.B. Dimethiconen vergleichbar sind. Wünschenswert war es insbesondere Rohstoffe zu Verfügung zu stellen, welche sich als Ersatzstoffe für Silikonöle eignen. Darüber hinaus war es von Interesse, Rohstoffe bereit zu stellen, die gegenüber den Rohstoffen des Standes der Technik eine verbesserte CO₂-Bilanz aufweisen.

Eine weitere Aufgabe bestand darin, Rohstoffe zur Verfügung zu stellen, welche eine stabile Formulierung mit AP/Deo (=Antiperspirant/Desodorant) Wirkstoffen ermöglicht. Kosmetische Zubereitungen der Kategorie Antiperspirantien/Desodorantien, insbesondere in so genannten "Stick-Formulierungen" haben immer noch das Problem der unzureichenden Stabilität der kosmetischen Grundlage. Hierbei ist u.a. die Härte der hergestellten "Stick-Formulierung" verbesserungsbedüftig. Nachteilig an bestehenden "Stick-Formulierung" ist, dass sich geruchliche Veränderungen während der Lagerung ergeben. Eine weitere Aufgabe der Erfindung bestand daher darin, Rohstoffe zu Verfügung zu stellen, welche es ermöglichen antiperspirierende bzw. desodorierende Zubereitungen, insbesondere solche in "Stick-Formulierung" stabil bereit zu stellen. Diese Zubereitungen sollten insbesondere bei längerer Lagerung keine unerwünschten Geruchsentwicklungen zeigen. Eine weitere Aufgabe bestand darin Rohstoffe zu Verfügung zu stellen, welche einen sensorisch "leichten" Eindruck vermitteln, möglichst bei gleichzeigter verbesserter Hautverträglichkeit, insbesondere in Kombination mit UV-Lichtschutzfiltern sowie in Verbindung mit Selbstbräunern. Von besonderem Interesse ist die Bereitstellung von neuen Rohstoffen, die in Formulierungen der dekorativen Kosmetik einen sensorisch vorteilhaften Eindruck ermöglichen. An Formulierungen der dekorativen Kosmetik, wie beispielsweise Lippenstifte, Lidschatten, Mascara, Nagellack etc. werden aufgrund des Applikationsorts (hauptsächlich Gesicht und Hände) erhöhte Anforderungen an die Sensorik, insbesondere an die Flüchtigkeit gestellt, damit diese Produkte nicht den Eindruck von "Schwere" vermitteln. Desweiteren ist bei diesen Produkten eine gute Dispergierbarkeit von Pigmenten wünschenswert.

Eine weitere Aufgabe bestand darin Rohstoffe zu Verfügung zu stellen, die sich als Filmbildner, insbesondere in haarkosmetischen Zubereitungen eignen.

### Beschreibung der Erfindung

Ein Gegenstand der Erfindung sind verzweigte Kohlenwasserstoffe, erhältlich durch Dehydroxymethylierung von Edukten ausgewählt aus der Gruppe bestehend aus
a) **verzweigten Alkoholen** der allgemeinen Formel R₁-OH, wobei R₁ für einen verzweigten Alkyl und/oder Alkenylrest mit 10 bis 29 C Atomen steht,
b) **verzweigten primären Diolen** der allgemeinen Formel HO-X-OH, wobei X für einen verzweigten Alkyl und/oder Alkenylrest mit 11 bis 44 C Atomen steht
c) **verzweigten primären Triolen** mit 12 bis 66 C Atomen.

Als Kohlenwasserstoffe werden organische Verbindungen bezeichnet, die nur aus Kohlenstoff und Wasserstoff bestehen. Sie umfassen sowohl cyclische als auch acyclische (=aliphatische) Verbindungen. Sie umfassen sowohl gesättigte wie einfach oder mehrfach ungesättigte Verbindungen. Die Kohlenwasserstoffe der vorliegenden Erfindung sind verzweigt, d.h. mindestens ein Kohlenstoffatom ist mit mehr als 2 Kohlenstoffatomen verbunden.

Der Begriff "verweigte Kohlenwasserstoffe" umfasst dabei sowohl einzelne verzweigte Kohlenswasserstoffe als auch beliebige Gemische der verzweigten Kohlenwasserstoffe.

In einer bevorzugten Ausführungsform der Erfindung enthalten die verzweigte Kohlenwasserstoffe kleiner gleich 20 Gew.-%, insbesondere kleiner gleich 10 Gew.-%, insbesondere kleiner gleich 5 Gew.-%, bevorzugt kleiner gleich 1 Gew.-%, **ungesättigte verzweigte Kohlenwasserstoffe** bezogen auf die Summe der verzweigten Kohlenwasserstoffe. In einer bevorzugten Ausführungsform der Erfindung enthalten die verzweigten Kohlenwasserstoffe kleiner gleich 0,1, insbesondere kleiner gleich 0,01 Gew.-%, insbesondere kleiner gleich 0,001 Gew.-% ungesättigte verzweigte Kohlenwasserstoffe bezogen auf die Summe der verzweigten Kohlenwasserstoffe.

In einer bevorzugten Ausführungsform der Erfindung weisen die verzweigten Kohlenwasserstoffe einen Anteil der ¹⁴C Isotope zu den ¹²C Isotopen von größer gleich 1x10⁻⁶, insbesondere bei größer gleich 1x10⁻¹⁵, vorzugsweise größer gleich 7,5x10⁻¹⁴, vorzugsweise größer gleich 1,5x10⁻¹³ , insbesondere größer gleich 3x10⁻¹³, vorzugsweise im Bereich von 6x10⁻¹³ bis 1,2x10⁻¹² auf. Bezugsgröße sind alle verzweigten Kohlenwasserstoffe.

Dies wird üblicherweise dadurch erreicht, dass die Edukte die entsprechenden Anteile an ¹⁴C Isotopen zu ¹²C Isotopen aufweisen.

Der Begriff Nuklide bezeichnet Atomarten (Atome einschließlich Elektronenhülle), die charakterisiert sind durch die Anzahl der Protonen und Neutronen im Kern, d.h. durch Ordnungszahl und Massenzahl (Zahl der Neutronen = Massenzahl - Ordnungszahl). Isotope sind Nuklide gleicher Ordnungszahl (= Kernladungszahl; Atomnummer, Protonenzahl), aber unterschiedlicher Anzahl der im Kern enthaltenen Neutronen und damit unterschiedlicher Massenzahl [=Nukleonenzahl, Zahl der in einem Atomkern enthaltenen Nukleonen (Protonen und Neutronen)]. Isotope unterscheiden sich außer durch die Masse auch durch Drehimpuls (Kernspin), magnetisches Moment und elektrisches Quadrupolmoment.

Zur eindeutigen Kennzeichnung der Isotope benutzt man die für Nuklide allgemein gebräuchliche Schreibweise ^{A}_{Z}X (X = chemisches Symbol, A = Massenzahl, Z = Kernladungszahl), für die stabilen Isotope des Kohlenstoff also ¹²₆C, alternativ wird als Schreibweise ¹²C oder C-12 verwendet.

Das Element Kohlenstoff hat insgesamt 2 stabile Isotope ¹²C und ¹³C. ¹²C kommt zu 98,9 % in der Natur vor, ¹³C zu 1,1 %. Neben diesen beiden stabilen Isotopen gibt es noch mehrere instabile Isotope. Das bekannteste instabile Isotop ist dabei ¹⁴C mit einer Halbwertszeit von 5730 Jahren. ¹⁴C entsteht durch natürliche Kernreaktion in der Atmosphäre aus ¹⁴N: die Erde ist ständig kosmischer Strahlung ausgesetzt, trifft diese Strahlung auf die obersten Schichten der Erdatmosphäre erzeugt dies freie Neutronen.

Diese wiederum reagieren mit dem in der unteren Atmosphäre zu etwa 80% in der Luft enthaltenen Stickstoff. Dabei läuft folgende Reaktion ab:

¹⁴N + ¹n → ¹⁴C + ¹p

Der Kern eines Stickstoffatoms mit der Massezahl 14 (7 Neutronen, 7 Protonen) nimmt ein Neutron auf. Unter Abgabe eines Protons entsteht aus dem Stickstoffatom das radioaktive Kohlenstoffisotop ¹⁴C (8 Neutronen, 6 Protonen), die Massezahl bleibt also gleich. Kohlenstoff ¹²C hat dagegen 6 Neutronen und 6 Protonen - ist also leichter als ¹⁴C.

Der in der Atmosphäre erzeugte ¹⁴C verbindet sich mit vorhandenem Sauerstoff zu Kohlendioxid. Durch die Photosynthese der Pflanzen gelangt ¹⁴C so anschließend in die Biosphäre. Da Lebewesen bei ihrem Stoffwechsel ständig Kohlenstoff mit der Atmosphäre austauschen, stellt sich in lebenden Organismen dasselbe Verteilungsverhältnis der 3 Kohlenstoff-Isotope ¹²C-12, ¹³C-12 und ¹⁴C ein, wie es in der Atmosphäre vorliegt: Lebende Organismen enthalten pro 10¹² stabilen ¹²C- u. ¹³C-Isotopen ca. 1,2 radioaktive ¹⁴C-Isotope.

Wird Kohlenstoff aus diesem Kreislauf herausgenommen (d.h. wird er fossil), dann ändert sich das Verhältnis zwischen ¹⁴C und ¹²C, da die zerfallenden 1¹⁴C Isotope nicht durch neue ersetzt werden.

Fossile Brennstoffe, wie Erdöl, Erdgas oder Kohle, sind vor über 100 Millionen Jahren entstanden, d.h. diese Brennstoffe enthalten keine ¹⁴C Isotope mehr, da die ursprünglich vorhandenen ¹⁴C Isotope zerfallen sind und keine neuen ¹⁴C Isotope aufgenommen wurden. Demnach enthalten Kohlenwasserstoffe, die aus fossilen Quellen stammen, keine ¹⁴C Isotope.

Der ¹⁴C-Gehalt einer Probe kann entweder durch Zählung der zerfallenden ¹⁴C-Isotope im Zählrohr (Zählrohr Methode nach Libby), im Flüssigkeits-Szintillations-Spektrometer oder durch Zählung der noch vorhandenen ¹⁴C-Isotope mit der Beschleuniger-Massenspektrometrie bestimmt werden. Mit der Beschleuniger-Massenspektrometrie (Abkürzung: AMS von *A*ccelerator *M*ass *S*pectrometry) können ¹⁴C Isotope mit Hilfe kernphysikalischer Meßmethoden im ppt- bis ppq-Bereich (von 10⁻¹² bis 10⁻¹⁶) in kleinsten Probenmengen (Milligrammbereich) nachgewiesen werden.

### Reduktive Dehydroxymethylierung

Durch die reduktive Dehydroxymethylierung wird die C-Kette des Eduktes pro OH-Gruppe um ein C-Atom verkürzt. Daher enthält der entstehende verzweigte Kohlenwasserstoff 1 C Atom weniger als das Edukt, falls das eingesetzte Edukt ein Alkohol gemäß a) ist, 2 C Atome weniger als das Edukt, falls das eingesetzte Edukte ein Diole gemäß b) ist, und 3 C Atome weniger als das Edukt, falls das Edukt ein Triol c) ist.

Der Begriff "verzweigt" im Sinne der vorliegenden Erfindung beschreibt Moleküle, die eine Hauptkette enthalten (= Kette mit der größten Anzahl an C Atomen) und mindestens eine Seitenkette (auch Verzweigung genannt). Daher enthalten die verzweigten Verbindungen der vorliegenden Erfindung (Edukte sowie Produkte) mindestens eine Kohlenstoffatom, welche mit 2 weiteren Kohlenstoffatomen verbunden ist (= Verzweigungsstelle).

Die erfindungsgemäßen verzweigten Kohlenwasserstoffe sind erhältlich durch reduktive Dehydroxymethylierung, wie beispielsweise in WO 2007/068371 beschrieben.

In einer bevorzugten Ausführungsform der Erfindung werden die verzweigten Kohlenwasserstoffe erhalten, indem man die reduktive Dehydroxymethylierung bei Temperaturen von 180 bis 300°C, vorzugsweise 200 bis 280°C und insbesondere von 220 bis 260°C durchführt.

In einer bevorzugten Ausführungsform der Erfindung werden die verzweigten Kohlenwasserstoffe erhalten, indem man die Dehydroxymethylierung bei Drücken von 2 bis 300 bar, insbesondere von 2 bis 250 bar, insbesondere von 5 bis 100 bar, vorzugsweise von 5 bis 80 bar und insbesondere von 10 bis 50 bar durchführt.

In einer bevorzugten Ausführungsform der Erfindung werden die verzweigten Kohlenwasserstoffe erhalten, indem man die Dehydroxymethylierung in Gegenwart eines Katalysators durchführt, insbesondere eines Platin Rubidium oder Nickel-haltigen Katalysators. Vorzugsweise werden Nickel-Katalysatoren verwendet und insbesondere handelsübliche Ni-haltige Hydrierkatalysatoren, wie z.B. Katalysatoren der Fa. Engelhard oder Kata Leuna. Die Katalysatoren können hierbei sowohl als Suspensionskatalysatoren für ein Semi-Batch - Verfahren, als auch als Festbettkatalysatoren für ein kontinuierliches Verfahren eingesetzt werden. Die Katalysatoren sind vorzugsweise im Mengen von 0, 1 bis 3 Gew.-%, bezogen auf die Menge an Edukten in der Reaktionsmischung vorhanden. Bevorzugt können auch Katalysatoren in Mengen von 0,2 bis 2 Gew.-% und insbesondere in Mengen von 0,5 bis 1,0 Gew.-% Verwendung finden. Bei einem Suspensionsverfahren hat sich eine Katalysatorkonzentration von 0,1 - 2 Gew.-% ig bezüglich auf die eingesetzte Menge an Edukte als geeignet erwiesen, wobei der bevorzugte Bereich bei 0,5 -1,0 Gew.-% Ni liegt.

Die Reaktion der Edukte zu den verzweigten Kohlenwasserstoffen erfolgt vorteilhafterweise unter Zugabe von Wasserstoff. Die Reaktion der Edukte zu den verzweigten Kohlenwasserstoffen erfolgt vorteilhafterweise unter gleichzeitiger Wasserentfernung.

Es hat sich als vorteilhaft erwiesen, Wasserstoff zu dem mit suspendiertem Katalysator vorgelegtem Alkohol zu dosieren und gleichzeitig entstehendes Reaktionswasser bzw. Reaktionsgase aus dem Reaktor zu entfernen. Bei einem kontinuierlichen Verfahren kann diese Wasserauskreisauskreisung beispielsweise in einem mehrstufigen Verfahren erfolgen. Das entstandene Reaktionsgemisch muss anschließend zur Katalysatorabtrennung filtriert werden. Danach wird zur Entfernung des Resteduktgehaltes bzw. von Spuren dimerer Reaktionsprodukte eine fraktionierte Destillation durchgeführt. Das hierbei erhaltene Sumpfprodukt kann für den nächsten Ansatz recyclisiert werden.

Anschließend kann zur Geruchsverbesserung noch eine Desodorierung nachgeschaltet werden.

In einer bevorzugten Ausführungsform der Erfindung werden die verzweigten Kohlenwasserstoffe erhalten, indem man die reduktive Dehydroxymethylierung in Gegenwart von Wasserstoff, einem Katalysator und bei Temperaturen von 100 bis 300°C und Drücken von 2 bis 300 bar durchführt, wobei während der Reaktion entstehendes Wasser entfernt wird.

Aus den erfindungsgemäß erhaltenen Reaktionsgemischen können, vorzugsweise nach Aufreinigung der Rohprodukte, z.B. nach fraktionierter Destillation und weiterhin vorzugsweise nachfolgender Desodorierung, hochreine verzweigte Kohlenwasserstoffe mit definierter Kettenlänge gewonnen werden. Die so erhaltenen verzweigten Kohlenwasserstoffe definierter Kettenlänge können entweder als Einzelkomponenten in kosmetischen Formulierungen verwendet werden oder definiert gemischt werden, um spezielle Eigenschaften, wie z.B. Spreitverhalten, Flüchtigkeit oder auch einen Flammpunkt einstellen zu können.

Die Erfindung umfasst auch verzweigte Kohlenwasserstoffe die durch Umsetzung beliebiger Mischungen der genannte Edukte a) bis c) erhalten werden.

### Edukte a) verzweigte Alkohole

In einer Ausführungsform der Erfindung werden als Edukte **verzweigte Alkohole** der allgemeinen Formel R₁-OH eingesetzt, wobei R₁ für einen verzweigten Alkyl und/oder Alkenylrest mit 10 bis 29 C Atomen, vorzugsweise 11 bis 20 C Atomen, und insbesondere mit 12 bis 18 C Atomen steht.

In einer bevorzugten Ausführungsform der Erfindung werden als verzweigte Alkohole primäre Alkohole eingsetzt.

In einer bevorzugten Ausführungsform der Erfindung werden als verzweigte Alkohole gesättigte Alkohole eingesetzt.

Prinzipiell kommen als Edukte sowohl einfach als auch mehrfach verweigte Alkohole in Frage. In einer bevorzugten Ausführungsform der Erfindung werden als verzweigte Alkohole einfach verzweigte Alkohole eingesetzt.
Die Seitenketten (= Verzweigung) ist üblicherweise kleiner gleich 5 C Atome, vorzugsweise kleiner gleich 4 C-Atome, vorzugsweise kleiner gleich 2 C-Atome. Besonders bevorzugt ist die Seitenkette eine Methylgruppe (= 1 C-Atom).

Prinzipiell kann der Alkohol die Verzweigung(en) an jeder Stelle der Hauptkette tragen. Besonders bevorzugt sind verzweigte Alkohole, welche die Verzweigung im mittleren Drittel der Hauptkette tragen. D.h. wenn als Alkohol beispielsweise ein verzweigter Alkohol mit einer Hauptkette von 15 C-Atomen und einer Seitenkette von 3 C-Atomen verwendet ist, so ist es vorteilhaft dass die Verzweigung sich in der Hauptkette zwischen dem 5. und dem 10. C-Atom befindet.

Geeignete verzweigte Alkohole sind beispielsweise die kommerziell erhältlichen Alkohole Isoundecanol (Exxal® 11), Isotridecanol (Exxal® 13). Isotridecanole (Isotridecylalkohole) werden beispielsweise durch Hydroformylierung aus gereinigtem Tetrapropen (Tetrapropylen) mit anschließender Hydrierung hergestellt. Diese Isotridecanole sind somit ein Gemisch von primären Isotridecanolen, bevorzugt von Tetramethylnonanolen.

Besonders bevorzugt als Edukt ist Isostearylalkohol. Isostearylalkohol sind beispielweise unter dem Handelsnamen Prisorine 3515 Isostearyl alcohol von der Fa. Unicema, Gouda, Niederlande erhältlich oder unter dem Handelsnamen Speziol 935 G von der Fa. Cognis GmbH, Düsseldorf.

Besonders geeignet sind Alkohole, welche durch Spaltung aus pflanzlichen Triglyceriden erhalten werden.

In einer bevorzugten Ausführungsform der Erfindung werden als Edukte verzweigte Alkohole eingesetzt, die einen Anteil an ¹⁴C Isotope zu den ¹²C Isotopen von größer gleich 1x10⁻¹⁶, aufweisen, insbesondere größer gleich 1x10⁻¹⁵, vorzugsweise größer gleich 7,5x10⁻¹⁴, vorzugsweise größer gleich 1,5x10⁻¹³ , insbesondere größer gleich 3x10⁻¹³, vorzugsweise im Bereich von 6x10⁻¹³ bis 1,2x10⁻¹². Bezugsgröße sind alle verzweigten Alkohole.

### Edukte b) verzweigte primäre Diole

In einer Ausführungsform der Erfindung werden als Edukte verzweigte primäre Diole der allgemeinen Formel HO-X-OH eingesetzt, wobei X für einen verzweigten Alkyl und/oder Alkenylrest mit 11 bis 44 C Atomen, vorzugsweise 13 bis 36 C Atomen, steht. Die verzweigten primären Diole tragen die OH-Gruppen jeweils endständig, d.h. alle 2 OH-Gruppen sind primäre OH Gruppen, es handelt sich also um alpha-omega-Diole.

In einer bevorzugten Ausführungsform der Erfindung werden als verzweigte primäre Diole gesättigte Diole eingesetzt.

Prinzipiell kommen als Edukte sowohl einfach als auch mehrfach verweigte primäre Diole in Frage. In einer bevorzugten Ausführungsform der Erfindung werden als verzweigte primäre Diole zweifach verzweigte primäre Diole eingesetzt.

Prinzipiell kann der das Diol die Verzweigung(en) an jeder Stelle der Hauptkette tragen. Besonders bevorzugt sind verzweigte Diole, welche die Verzweigung im mittleren Drittel der Hauptkette tragen. D.h. wenn als Diol beispielsweise ein verzweigtes Diol mit einer Hauptkette von 15 C-Atomen und zwei Seitenketten von je 3 C-Atomen verwendet wird, so ist es vorteilhaft dass die Verzweigungen sich in der Hauptkette zwischen dem 5. und dem 10. C-Atom befinden.

Ein geeignetes verzweigte primäres Diole ist beispielsweise das Dimerdiol α,ω C36 (= alpha,omega C36 Diol), das beispielsweise unter dem Handelsnamen Speziol C 36/2 der Fa. Cognis GmbH, Düsseldorf erhältlich ist.

In einer bevorzugten Ausführungsform der Erfindung werden als Edukte verzweigte primäre Diole eingesetzt, die einen Anteil an ¹⁴C Isotope zu den ¹²C Isotopen von größer gleich 1x10⁻¹⁶ , aufweisen, insbesondere größer gleich 1x10⁻¹⁵, vorzugsweise größer gleich 7,5x10⁻¹⁴, vorzugsweise größer gleich 1,5x10⁻¹³ , insbesondere größer gleich 3x10⁻¹³, vorzugsweise im Bereich von 6x10⁻¹³ bis 1,2x10⁻¹². Bezugsgröße sind alle verzweigten primären Diole.

### Edukte c) verzweigte primäre Triole

In einer Ausführungsform der Erfindung werden als Edukte **verzweigte primäre Triole** mit 12 bis 66 C Atomen, vorzugsweise 14 bis 54 C Atomen eingesetzt. Die verzweigten primären Triole tragen die OH-Gruppen jeweils endständig, d.h. alle 3 OH-Gruppen sind primäre OH Gruppen.

In einer bevorzugten Ausführungsform der Erfindung werden als verzweigte primäre Triole **gesättigte** Triole eingesetzt.

Prinzipiell kommen als Edukte sowohl einfach als auch mehrfach verweigte primäre Triole in Frage. In einer bevorzugten Ausführungsform der Erfindung werden als verzweigte primäre Triole mehrfach verzweigte, vorzugsweise zweifach verzweigten primäre Triole eingesetzt.

Ein geeignetes verzweigtes primäres Triol ist beispielsweise das Trimertriol, das beispielsweise unter dem Handelsnamen Sovermol 650NS der Fa. Cognis GmbH, Düsseldorf erhältlich ist.

In einer bevorzugten Ausführungsform der Erfindung werden als Edukte verzweigte primäre Triole eingesetzt, die einen Anteil an ¹⁴C Isotope zu den ¹²C Isotopen von größer gleich 1x10⁻¹⁶, aufweisen, insbesondere größer gleich 1x10⁻¹⁵, vorzugsweise größer gleich 7,5x10⁻¹⁴, vorzugsweise größer gleich 1,5x10⁻¹³, insbesondere größer gleich 3x10⁻¹³, vorzugsweise im Bereich von 6x10⁻¹³ bis 1,2x10⁻¹². Bezugsgröße sind alle verzweigten primären Triole.

### Mischungen der Edukte b) und c)

Die Erfindung umfasst auch verzweigte Kohlenwasserstoffe die durch Umsetzung beliebiger Mischungen der genannte Edukte a) bis c) erhalten werden. Besonders bevorzugt sind verzweigte Kohlenwasserstoffe, die durch Umsetzung von Mischungen aus primären verzweigten Diolen und primären verzweigten Triolen erhalten werden.

Als primäre ungesättigte Diole sowie als primäre ungesättigte Triole können bevorzugt technische Gemische eingesetzt werden, die durch Oligomerisierung von ungesättigten Fettsäuren mit 12 bis 22, vorzugsweise 16 bis 18 Kohlenstoffatomen oder deren Methylester und nachfolgende Hochdruckhydrierung erhalten werden.

Die Oligomerisierung von ungesättigten Fettsäuren stellt eine bekannte elektrozyklische Reaktion dar. Bei der Oligomerisierung treten durchschnittlich zwei bis drei Fettsäuren zusammen und bilden Dimere bzw. Trimere, die überwiegend cycloaliphatische Strukturen aufweisen. Neben der Fraktion der Dimeren und Trimeren wird eine sogenannte Monomerfraktion erhalten, in der sich nicht umgesetzte Ausgangsstoffe und verzweigte Monomere befinden, die im Verlauf der Reaktion durch Isomerisierung entstanden sind. Daneben gibt es selbstverständlich auch eine Fraktion höherer Oligomeren, die jedoch in der Regel nicht von größerer Bedeutung ist. Die Oligomerisierung kann thermisch oder in Gegenwart von Edelmetallkatalysatoren durchgeführt werden. Vorzugsweise erfolgt die Reaktion in Gegenwart von Tonerden wie beispielsweise. Die Regelung des Gehaltes an Dimeren und Trimeren bzw. der Umfang der Monomerfraktion kann durch die Reaktionsbedingungen gesteuert werden.

Als Ausgangsstoffe für die Oligomerisierung kommen beispielsweise technische ungesättigte Fettsäuren mit 12 bis 22 und vorzugsweise 16 bis 18 Kohlenstoffatomen in Betracht. Typische Beispiele sind Palmoleinsäure, Ölsäure, Elaidylsäure, Petroselinylsäure, Linolsäure, Linolensäure, Konjuenfettsäure, ElaeoStearinsäure, Ricinolsäure, Gadoleinsäure, Erucasäure sowie deren technische Gemische mit gesättigten Fettsaeuren. Typische Beispiele für geeignete technische Gemische sind ungehärtete Spaltfettsäuren natürlicher Triglyceride mit Iodzahlen im Bereich von 40 bis 140, wie etwa Palmfettsäure, Talgfettsäure, Rübölfettsäuren, Sonnenblumenfettsäure und dergleichen. Bevorzugt sind Spaltfettsäuren mit einem hohen Gehalt an Ölsäure.

Neben den Fettsäuren können auch deren Ester, vorzugsweise Methylester dimerisiert werden. Es ist gleichfalls möglich, die Säure zu oligomerisieren und vor der Hydrierung in die Methylester zu überführen. Die Hydrierung der Säure- bzw. Ester- zur Alkoholgruppe gelingt in an sich bekannter Weise in Gegenwart heterogener Kupfer/Chrom- oder Kupfer/Zink-Katalysatoren bei Temperaturen im Bereich von 200 bis 300 °C und Drücken von etwa 250 bis 350 bar.

Primäre ungesättigte Diole sowie primäre ungesättigte Triole, die im Sinne der Erfindung besonders bevorzugt sind, werden durch Oligomerisierung von technischer Ölsäure und nachfolgende Hochdruckhydrierung erhalten werden und weisen einen Dimerdiolgehalt von 33 bis 99 Gew.-% sowie einen Trimertriolgehalt von 1 bis 67 Gew.-% auf. Der Gehalt an Monomeren kann 1 bis 15 Gew.-% betragen und falls erforderlich durch Destillation erniedrigt werden.

Die erfindungsgemäßen verzweigten Kohlenwasserstoffe eignen sich insbesondere zur Verwendung in kosmetischen und/oder pharmazeutischen Zubereitungen, insbesondere als Ölkörper und/oder als Wachskomponenten.

Ein weiterer Gegenstand der Erfindung betrifft daher die Verwendung der erfindungsgemäßen verzweigten Kohlenwasserstoffen in kosmetischen und/oder pharmazeutischen Zubereitungen, insbesondere als Ölkörper und/oder als Wachskomponente.

Ein weiterer Gegenstand der Erfindung betrifft ein Verfahren zur Herstellung einer kosmetischen und/oder pharmazeutischen Zubereitung, wobei ein erfindungsgemäßer verzweigter Kohlenwasserstoff gemäß einem der Ansprüche 1 bis 11 zu einen kosmetisch und/oder pharmazeutisch geeigneten Träger gegeben wird.

Die erfindungsgemäßen verzweigten Kohlenwasserstoffe eignen sich insbesondere zur Verwendung in kosmetischen Zubereitungen zur Pflege von Haut und/oder Haaren.

Die erfindungsgemäßen verzweigten Kohlenwasserstoffe eignen sich insbesondere zur Verwendung in kosmetischen Zubereitungen zum Sonnenschutz.

Die erfindungsgemäßen verzweigten Kohlenwasserstoffe eignen sich insbesondere zur Verwendung in Zubereitungen der dekorativen Kosmetik, wie beispielsweise Lippenstifte, Lip Gloss, Lidschatten, Wimperntuschen (Mascara), Lidstifte (Kajal), Nagellack sowie in Make-up Formulierungen jeder Art (Puder, Creme, Foundation, Abdeckstifte etc.). Die erfindungsgemäßen verzweigten Kohlenwasserstoffe eignen sich insbesondere zur Verwendung in Zubereitungen zur Reinigung von Haut und/oder Haaren, wie beispielsweise Shampoos, Duschgels, Badezusätze, Conditioner etc.

Die erfindungsgemäßen verzweigten Kohlenwasserstoffe eignen sich weiterhin zur Herstellung von feinteiligen Emulsionen, so z.B. Nanoemulsionen, Microemulsionen oder PIT Emulsionen. In solchen feinteiligen Emulsionen liegen die Öltröpfchen in der Regel im Bereich von 10 bis 1000 nm, vorzugsweise 100 bis 500 nm Durchmesser vor. Diese werden nach dem Fachmann bekannten Verfahren hergestellt, für PIT Emulsionen beispielsweise in Parfümerie und Kosmetik, 77. Jahrgang, Nr. 4/96, S. 250 - 254 von Wadle et al. beschrieben.

### Kosmetische und/oder pharmazeutische Zubereitungen

Ein weiterer Gegenstand der Erfindung betrifft kosmetische und/oder pharmazeutische Zubereitungen, enthaltend 0,1 bis 80 Gew.%, vorzugsweise 5 bis 50 Gew.-%, insbesondere 10 bis 30 Gew.-% der erfindungsgemäßen verzweigten Kohlenwasserstoffe. Die Gew.-% beziehen sich auf das Gesamtgewicht der kosmetischen und/oder pharmazeutischen Zubereitung.

Vorzugsweise beträgt der Anteil der ¹⁴C Isotope zu den ¹²C Isotopen in dem in den erfindungsgemäßen Zubereitungen enthaltende verzweigten Kohlenwasserstoffe größer gleich 1 x 10⁻¹⁶, insbesondere größer gleich 1 x 10⁻¹⁵, vorzugsweise größer gleich 7,5 x 10⁻¹⁴ , vorzugsweise größer gleich 1,5 x 10⁻¹³, insbesondere größer gleich 3 x 10⁻¹³, vorzugsweise liegt der Anteil der ¹⁴C Isotope zu den ¹²C Isotopen im Bereich von 6 x 10⁻¹³ bis 1,2 x 10⁻¹². Bezugsgröße sind hier alle in der erfindungsgemäßen Zubereitung enthaltenen verzweigten Kohlenwasserstoffe.

In einer bevorzugten Ausführungsform enthalten die erfindungsgemäßen kosmetischen und/oder pharmazeutischen Zubereitungen verzweigte Kohlenwasserstoffe, die kleiner gleich 20 Gew.-%, insbesondere kleiner gleich 10 Gew.-%, kleiner gleich 5 Gew.%, vorzugsweise kleiner gleich 1 Gew.-% verzweigte **ungesättigte** Kohlenwasserstoffe bezogen auf die Summe der verzweigten Kohlenwasserstoffe enthalten.

In einer bevorzugten Ausführungsform der Erfindung enthalten die Zubereitungen kleiner gleich 0,1, insbesondere kleiner gleich 0,01 Gew.-%, insbesondere kleiner gleich 0,001 Gew.-% ungesättigte verzweigte Kohlenwasserstoffe bezogen auf die Summe der verzweigten Kohlenwasserstoffe.

Die erfindungsgemäßen kosmetischen und/oder pharmazeutischen Zubereitungen stellen leichte und stabile kosmetische und/oder pharmazeutische Zubereitungen dar, dies ist insbesondere dann der Fall, wenn sie weiterhin Antiperspirant-/Desodorant-Wirkstoffen enthalten.

Ein Gegenstand der Erfindung betrifft daher kosmetische und/oder pharmazeutische Zubereitungen, enthaltend 0,1 bis 80 Gew.-% der erfindungsgemäßen verzweigten Kohlenwasserstoffe und mindestens einen Antiperspirant-/ Desodorant-Wirkstoff.

Erfindungsgemäß sind als Antiperspirant/Desodorant Wirkstoff alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend Antiperspirantien, Esteraseinhibitoren, bakterizide bzw. bakteriostatische Wirkstoffe und/oder schweißabsorbierende Substanzen.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt.

Die erfindungsgemäßen Zubereitungen können die Antiperspirantien in Mengen von 1 bis 50, vorzugsweise 5 bis 30 und insbesondere 8 bis 25 Gew.-% - bezogen auf das Gesamtgewicht der kosmetischen und/oder pharmazeutischen Zubereitung - enthalten.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf das Gesamtgewicht der kosmetischen und/oder pharmazeutischen Zubereitung - enthalten.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.
Die erfindungsgemäßen Zubereitungen können die bakteriziden bzw. bakteriostatischen Wirkstoffe in Mengen von 0,01 bis 5 und vorzugsweise 0,1 bis 2 Gew.-% - bezogen auf das Gesamtgewicht der kosmetischen und/oder pharmazeutischen Zubereitung - enthalten.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf das Gesamtgewicht der kosmetischen und/oder pharmazeutischen Zubereitung - enthalten.

Die erfindungsgemäßen kosmetischen und/oder pharmazeutischen Zubereitungen stellen leichte und stabile kosmetische und/oder pharmazeutische Zubereitungen dar, dies ist insbesondere dann der Fall, wenn sie weiterhin mindestens einen UV-Lichtschutzfilter enthalten.

Ein Gegenstand der Erfindung betrifft daher kosmetische und/oder pharmazeutische Zubereitungen, enthaltend 0,1 bis 80 Gew.% der erfindungsgemäßen verzweigten Kohlenwasserstoffe, und mindestens mindestens einen UV-Lichtschutzfilter.

Erfindungsgemäß sind als UV-Lichtschutzfilter bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
➢ 3-(4'-Trimethylammonium) benzyliden- bornan-2-on-methylsulfat (Mexoryl SO)
➢ 3,3'-(1,4-Phenylendimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)- [2-oxoborn-3-yliden)methyl}benzyl]acrylamid (Mexoryl SW)
➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)aminocarbonyl) phenyl-amino]-1,3,5-triazin-2,4- diyl)diimino] bis(benzoesäure-2-ethylhexylester) (Uvasorb® HEB);
➢ 2,2(-Methylen-bis(6-(2H-benzotriazol-2-yl)-4- (1,1,3,3-tetramethyl-butyl)phenol) (Tinosorb M);
➢ 2,4-Bis[4-(2-ethylhexyloxy)-2- hydroxyphenyl]-6-(4- methoxyphenyl)-1,3,5- triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der Fassung Commission Directive 2005/9/EC of 28 January 2005 amending Council Directive 76/768/EEC, concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pig-mente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 8/1996, S. 543-548 **sowie** Parf.Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Ein Gegenstand der Erfindung betrifft daher kosmetische und/oder pharmazeutische Zubereitungen, enthaltend 0,1 bis 80 Gew.% der erfindungsgemäßen verzweigten Kohlenwasserstoffe, und mindestens mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden- bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy) disiloxanyl)propyl) phenol, Dimethicodiethylbenzalmalonate und ihren Mischungen.

Diese UV-Lichtschutzfilter sind beispielsweise unter den folgenden Handelsnamen kommerziell erhältlich:
NeoHeliopan®MBC (INCI: 4-Methylbenzylidene Camphor; Hersteller: Symrise); NeoHeliopan® BB (INCI: Benzophenone-3, Hersteller: Symrise); Parsol®1789 (INCI: Butyl Methoxydibenzoylmethane, Hersteller: Hoffmann-La Roche (Givaudan); Tinosorb®S (INCI: Bis-Ethylhexyloxyphenol Methoxyphenyl Triazine); Tinosorb®M (INCI: Methylene Bis-Benzotriazolyl Tetramethylbutylphenol): Herstelller: Ciba Specialty Chemicals Corporation; Uvasorb®HEB (TNCI: Diethylhexyl Butamido Triazone, Hersteller: 3V Inc.), Uvinul®T 150 (INCI: Ethylhexyl Triazone, Hersteller: BASF AG); Uvinul® A plus (INCI: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCI: Camphor Benzalkonium Methosulfate; Mexoryl®SX: 3,3'-(1,4-Phenylendimethin)-bis (7,7- dimethyl-2-oxobicyclo-[2.2.1] heptan-1-methansulfonsäure), CTFA: INCI Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCI Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy) disiloxanyl)propyl) phenol; INCI: DROMETRIZOLE TRISILOXANE; Parsol® SLX: Dimethicodiethylbenzalmalonate, INCI Polysilicone-15.

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf das Gesamtgewicht der kosmetischen und/oder pharmazeutischen Zubereitung - enthalten.

Ein Gegenstand der Erfindung betrifft kosmetische und/oder pharmazeutische Zubereitungen, enthaltend 0,1 bis 80 Gew.-% der erfindungsgemäßen verzweigten Kohlenwasserstoffe und mindestens einen Selbstbräuner.

Als Selbstbräuner sind Substanzen zu verstehen, welche eine Bräunung der Haut verursachen. Beispielsweise seien sowie alpha, beta-ungestättige Aldehyde genannt, welche mit den Aminosäuren der Haut im Sinne einer Maillard Reaktion zu gefärbten Verbindungen abreagieren. Als Wirkstoffe für Selbstbräuner kommen weiterhin in Frage natürliche oder synthetische Ketole oder Aldole. Als geeignete Wirkstoffe seien exemplarisch genannt Dihydroxyaceton, Erythrulose Glycerolaldehyd, Alloxan, Hydroxymethylglyoxal, gamma-Dialdehyd, 6-Aldo-D-Fructose, Ninhydrin und meso-Weinsäuredialdehyd. Als Selbstbräuner eignen sich insbesondere Dihydroxyaceton und/oder Erythrulose.

Als besonders vorteilhaft haben sich Mischungen der o.g. Wirkstoffe untereinander oder mit Mucondialdehyd oder/und Naphthochinone wie z. B. 5-Hydroxy-1,4-naphthochinon (Juglon) und 2-Hydroxy-1,4-Naphthochinon erwiesen.

Die erfindungsgemäßen Zusammensetzungen enthalten die Selbstbräuner üblicherweise in Konzentrationen von 1 bis 10, insbesondere von 2 bis 5 Gew.-% -bezogen auf das Gesamtgewicht der kosmetischen und/oder pharmazeutischen Zubereitung.

Ein Gegenstand der Erfindung betrifft kosmetische und/oder pharmazeutische Zubereitungen, enthaltend 0,1 bis 80 Gew.% der erfindungsgemäßen verzweigten Kohlenwasserstoffe und mindestens einen Selbstbräuner und mindestens einen UV-Lichtschutzfilter.

Die erfindungsgemäßen kosmetischen und/oder pharmazeutischen Zubereitungen können beispielsweise als O/W oder W/O Pflegeemulsionen, Sonnenschutzformulierung, AP/Deo Konzepte, Formulierungen für die dekorative Kosmetik, ölige Pflegezubereitungen, Tränkflüssigkeiten für Substrate, wie beispielsweise Papier- und Vliessprodukte vorliegen. Exemplarisch seien genannt Wet Wipes, Taschentücher, Windeln oder Hygieneprodukte.

Die erfindungsgemäßen verzweigten Kohlenwasserstoffe sowie die erfindungsgemäßen kosmetischen und/oder pharmazeutischen Zubereitungen, eignen sich insbesondere auch für leichte, sprühbare Anwendungen und/oder als Bestandteile von Pflegeemulsionen für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung. Sie lassen sich auf Tissues, Papiere, Wipes, Vlies-Produkte, Schwämme, Puffs, Pflaster und Bandagen applizieren, die ihren Einsatz im Bereich der Reinigung, Hygiene und/oder Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes, Toilettenpapier, Erfrischungstücher, After-shave Tücher). Sie lassen sich u.a. auch in Zubereitungen zur Haarpflege, Haarreinigung oder Haarfärbung einsetzen. Durch den Einsatz der erfindungsgemäßen verzweigten Kohlenwasserstoffe wird das sensorische Verhalten bei Applikation positiv beeinflusst

Die erfindungsgemäßen verzweigten Kohlenwasserstoffe eignen sich insbesondere als Bestandteile von Zubereitungen der dekorativen Kosmetik, wie beispielsweise Lippenstifte, Augen-Make up, wie beispielsweise Lidschatten, Mascara, Lidstifte, Kajal, Nagellack, etc. sowie Make-up Formulierungen.

Ein Gegenstand der Erfindung betrifft kosmetische und/oder pharmazeutische Zubereitungen, enthaltend 0,1 bis 80 Gew.-% der erfindungsgemäßen verzweigten Kohlenwasserstoffe, und mindestens ein Pigment und/oder einen Farbstoff.

Der Begriff Pigment umfasst Partikel jeder Form, die weiß oder gefärbt, organisch oder anorganisch sind, in den Zubereitungen nicht löslich sind, und dem Zweck dienen die Zubereitung zu färben. In einer bevorzugten Ausführungsform werden anorganische Pigmente verwendet, besonders bevorzugt sind Metalloxide.

Als anorganische Pigmente seien exemplarisch genannt: Titandioxid, optional oberflächenbeschichtet, Zirkonium oder Ceriumoxide und Zink-, Eisen- (Schwarz, Gelb oder Rot) und Chrom-oxide, Manganviolett, Ultramarine Blau, Chromhydroate und Eisen(III)blau, Metallpulver wie Aluminiumpulver oder Kupferpulver.

In einer bevorzugten Ausführungsform der Erfindung ist das Pigment ausgewählt aus den anorganischen Pigmenten, vorzugweise aus den Metalloxiden. In einer bevorzugten Ausführungsform ist das Pigment ausgewählt aus der Gruppe bestehend aus Titandioxid, Zinkoxid, Eisenoxid und Mischungen daraus. Die Pigmente können sowohl einzeln als auch in Mischungen vorliegen. Bevorzugt im Sinne der vorliegenden Erfindung sind Pigmentmischungen aus Weiss-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen koennen. Unter den Farbpigmenten sind Eisenoxide besonders bevorzugt.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäss vorteilhaft zusätzlich zu einem oder mehreren Weiss- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenfoermiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenfoermige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phaenomen der Interferenz (Interferenzpigmente) (DIN 55944: 2003-11).

Beispiele für erfindungsgemäss bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).

Ferner können die erfindungsgemässen Zubereitungen vorteilhaft auch organische Farbpigmente enthalten, d. h. organische Farbstoffe, welche in der Zubereitung praktisch unlöslich sind. Nach **DIN 55944: 1990-04** können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weisspigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen.
Die erfindungsgemäßen Zubereitungen enthalten üblicherweise 0,1 bis 40 Gew.-% Pigmente - bezogen auf das Gesamtgewicht der kosmetischen und/oder pharmazeutischen Zubereitung.

Es ist ferner vorteilhaft im Sinne der vorliegenden Erfindung, wenn die erfindungsgemässe Zubereitung ein oder mehrere Farbstoffe enthält.

Die Farbstoffe können sowohl synthetischen als auch natürlichen Ursprungs sein. Eine Liste von geeigneten Farbstoffen findet sich in EP 1371 359 A2**,** S.8, Z. 25-57, S.9 und S.10 sowie S.11, Z. 1 bis 54, auf welche hiermit explizit Bezug genommen wird.Die erfindungsgemäßen Zubereitungen enthalten üblicherweise 0,01 bis 5, vorzugsweise 0,1 bis 1,0 Gew.-% Farbstoffe- bezogen auf das Gesamtgewicht der kosmetischen und/oder pharmazeutischen Zubereitung. Die erfindungsgemäßen Zubereitungen enthalten üblicherweise eine Gesamtmenge an Farbstoffen und Pigmenten im Bereich von 0,01 bis 30 Gew.-%, insbesonderen 0,1 bis 15 Gew.-%, vorzugsweise 1 bis 10 Gew.-%, bezogen auf das Gesamtgewicht der kosmetischen und/oder pharmazeutischen Zubereitung.

Als Farbstoffe und Pigmente eignen sich insbesondere die gemäß Annex IV der Kommissions Direktive zugelassenen Farbstoffe und Pigmente (in der Fassung: Commission Directive 2007/22/EC of 17 April 2007 amending Council Directive 76/768/EEC**,** concerning cosmetic products, for the purposes of adapting Annexes IV and VI thereto to technical progress) zugelassen Stoffe, auf die hiermit explizit Bezug genommen wird.

Die kosmetischen und/oder pharmazeutischen Zubereitungen können Formulierungen zur Körperpflege sein, z. B. eine Körpermilch, Cremes, Lotionen, sprühbare Emulsionen, Produkte zur Eliminierung des Körpergeruchs etc. Die erfindungsgemäßen verzweigten Kohlenwasserstoffe lassen sich auch in tensidhaltigen Formulierungen wie z. B. Schaum- und Duschbädem, Haarshampoos und Pflegespülungen einsetzen. Je nach Applikationszweck enthalten die kosmetischen und/oder pharmazeutischen Zubereitungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Ein Gegenstand der Erfindung betrifft kosmetische und/oder pharmazeutische Zubereitungen, enthaltend 0,1 bis 80 Gew.-% der erfindungsgemäßen verzweigten Kohlenwasserstoffe und mindestens einen Emulgator und/oder ein Tensid und/oder eine Wachskomponente und/oder ein Polymer und/oder einen Ölkörper.

### Emulgator

In einer Ausführungsform der Erfindung enthalten die erfindungsgemäßen Zubereitungen mindestens einen Emulgator.

Ein Gegenstand der Erfindung betrifft kosmetische und/oder pharmazeutische Zubereitungen, enthaltend 0,1 bis 80 Gew.-% der erfindungsgemäßen verzweigten Kohlenwasserstoffe und mindestens einen Emulgator.

Die erfindungsgemäßen Zusammensetzungen enthalten den/die Emulgator(en) üblicherweise in einer Menge von 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zusammensetzung.

Jedem Emulgator wird ein sogenannter HLB-Wert (eine dimensionslose Zahl zwischen 0 und 20) zugeschrieben, der angibt, ob eine bevorzugte Wasser-oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Grösse und Stärke der hydrophilen und der lipophilen Gruppen eines Emulgators aus.

Der HLB-Wert eines Emulgators lässt sich auch aus Inkrementen errechnen, wobei die HLB-Inkremente für die verschiedenen hydrophilen und hydrophoben Gruppen, aus denen sich ein Molekül zusammensetzt. In der Regel kann er Tabellenwerken (z. B. H. P. Fiedler, Lexikon der Hilfsstoffe für Pharmazie, Kosmetik und angrenzende Gebiete, Editio Cantor Verlag, Aulendorf, 4. Aufl. 1996) oder den Herstellerangaben entnommen werden. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Öiphase entsteht unter sonst gleichen Herstellungsbedingungen eine W/O-Emulsion.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls^{®} PGPH" (W/O-Emulgator) oder "Eumulgin^{®} VL 75" (Abmischung mit Lauryl Glucosides im Gewichtsverhältnis 1:1, O/W-Emulgator) oder Dehymuls^{®} SBL (W/O-Emulgator) von der Cognis Deutschland GmbH vertrieben werden. In diesem Zusammenhang sei insbesondere auf das Europäische Patent EP 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Besonders bevorzugte Emulgatoren sind z. B. Cetyl Dimethicone Copolyol (z.B. Abil EM-90), Polyglyceryl-2 Dipolyhydroxystearate (z.B. Dehymuls PGPH), Polyglycerin-3-Diisostearate (z.B. Lameform TGI), Polyglyceryl-4 Isostearate (z.B. Isolan GI 34), Polyglyceryl-3 Oleate (z.B. Isolan GO 33), Diisostearoyl Polyglyceryl-3 Diisostearate (z.B. Isolan PDI), Polyglyceryl-3 Methylglucose Distearate (z.B. Tego Care 450), Polyglyceryl-3 Beeswax (z.B. Cera Bellina), Polyglyceryl-4 Caprate (z.B. Polyglycerol Caprate T2010/90), Polyglyceryl-3 Cetyl Ether (z.B. Chimexane NL), Polyglyceryl-3 Distearate (z. B. Cremophor GS 32) und Polyglyceryl Polyricinoleate (z.B. Admul WOL 1403), Glyceryl Oleate (z.B. Monomuls 90-O 18), Alkyl Glucoside (z.B. Plantacare 1200, Emulgade PL 68/50, Montanov 68, Tego Care CG 90, Tego Glucosid L 55), Methyl Glucose Isostearate (z.B. Tego Care IS), Methyl Glucose Sesquistearate (Tego Care PS), Sodium Cocoyl Hydrolyzed Wheat Protein (z.B. Gluadin WK), Potassium Cetyl Phosphate (z.B. Amphisol K, Crodafos CKP), Sodium Alkylsulfate (z.B. Lanette E), Sucrose Ester (z.B. Crodesta F-10, F-20, F-50, F-70, F-110, F-160, SL-40, Emulgade® Sucro), ethoxylierte und/oder propoxylierte Fettalkohole Fettsäuren, Rizinusöle bzw. hydrierte Rizinusöle (z.B. Eumulgin B2, B2, B3, L, HRE 40, HRE 60, RO 40, Cremophor HRE 40, HRE 60, L, WO 7, Dehymuls HRE 7, Arlacel 989), PEG-30. Dipolyhydroxystearate (z.B. Arlacel P 135, Dehymuls LE), Sorbitan Ester, Sorbitan Ester ethoxyliert und/oder propoxyliert sowie deren Gemische. Ein besonders effektives Gemisch besteht aus Polyglyceryl-2 Dipolyhydroxystearate und Lauryl Glucoside und Glycerin (z.B. Eumulgin VL 75). Geeignet sind weiterhin Polyglyceryl-4 Diisostearate/Polyhydroxystearate/Sebacate (Isolan^{®} GPS), Diisostearoyl Polyglyceryl-3 Diisostearate (z. B. Isolan PDI), Alkalisalze Acylglutamate (z.B. Eumulgin SG).

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer,"Encyclopedia of Chemical Technology", 3. Aufl., 1979, Band 8, Seite 913**,** aufgelistet. Für ethoxylierte Produkte lässt sich der HLB-Wert auch nach folgender Formel berechnen: HLB = (100 - L) : 5, wobei L der Gewichtsanteil der lipophilen Gruppen, d.h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitan-dihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.
Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur: 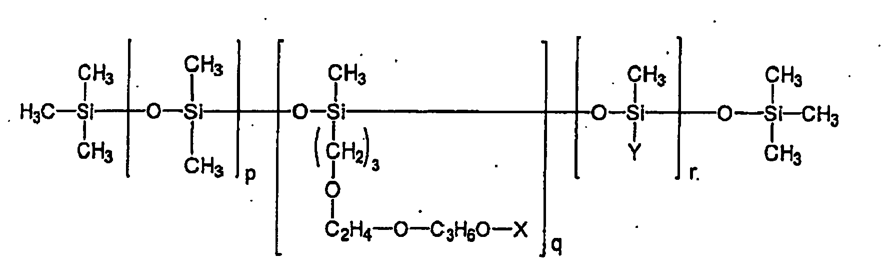 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden.

Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikGoldschmidt unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von Evonik Goldschmidt unter der Warenbezeichnung ABIL®EM 97 und ABIL®WE 09 verkauft wird.

Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist.

Ein weiterer vorteilhafter Silikonemulgator ist, Octyl Dimethicon Ethoxy Glucosid der Firma Wacker.
Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z. B. ABIL® EM 90 Evonik Goldschmidt), DC5200 Formulation Aid (Dow Corning) ] sowie beliebige Mischungen aus beiden Emulgatoren.

### Tenside

In einer Ausführungsform der Erfindung enthalten die erfindungsgemäßen Zubereitungen mindestens ein Tensid.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekuelteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Als Tenside werden üblicherweise oberflächen aktive Substanzen verstanden, die einen HLB-Wert von größer 20 aufweisen.

Ein Gegenstand der Erfindung betrifft kosmetische und/oder pharmazeutische Zubereitungen, enthaltend 0,1 bis 80 Gew.-% der erfindungsgemäßen verzweigten Kohlenwasserstoffe und mindestens ein Tensid.

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Die erfindungsgemäßen Zusammensetzungen enthalten den/die Tenside üblicherweise in einer Menge von 0 bis 40 Gew.-%., vorzugsweise 0,05 bis 30 Gew.-% insbesondere 0,05 bis 20 Gew.-%, vorzugsweise 0,1 bis 15 Gew.-% und insbesondere 0,1 bis 10 Gew.-% bezogen auf das Gesamtgewicht der Zusammensetzung.

Typische Beispiele für nichtionische Tenside sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine -COO⁽⁻⁾-oder -SO₃⁽⁻⁾ -Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈ -Acylsarcosin.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

Anionische Tenside sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zusammensetzungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

### Wachskomponente

In einer Ausführungsform der Erfindung enthalten die erfindungsgemäßen Zubereitungen mindestens eine Wachskomponente. In einer Ausführungsform der Erfindung enthalten die erfindungsgemäßen Zubereitungen die erfindungsgemäßen verzweigten Kohlenwasserstoffe als Wachskomponente. In der hier als bevorzugte genannten Ausführungsform enthalten die Zubereitungen somit eine von dem erfindungsgemäßen verzweigten Kohlenwasserstoffen verschiedene Wachskomponente, auch als "weitere Wachskomponente" bezeichnet.

In einer Ausführungsform der Erfindung enthalten die erfindungsgemäßen Zubereitungen mindestens eine (weitere) Wachskomponente.

Ein Gegenstand der Erfindung betrifft kosmetische und/oder pharmazeutische Zubereitungen, enthaltend 0,1 bis 80 Gew.-% der erfindungsgemäßen verzweigten Kohlenwasserstoffe und mindestens eine Wachskomponente.

Die erfindungsgemäßen Zubereitungen enthalten den/die (weiteren) Wachskomponente(n) üblicherweise in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zubereitung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 °C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten Triglycerid-Gemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 18 (C18-Alkohol), Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zusammensetzungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oder -stearat kommen hierfür in Frage.

Als Wachse eignen sich weiterhin Perlglanzwachse. Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Polymere

In einer Ausführungsform der Erfindung enthalten die erfindungsgemäßen Zubereitungen mindestens ein Polymer.

Ein Gegenstand der Erfindung betrifft kosmetische und/oder pharmazeutische Zubereitungen, enthaltend 0,1 bis 80 Gew.% der erfindungsgemäßen verzweigten Kohlenwasserstoffe und mindestens ein Polymer.

Die erfindungsgemäßen Zubereitungen enthalten das/die Polymere(n) üblicherweise in einer Menge von 0 bis 20 Gew.-%, vorzugsweise 0,1 bis 15 Gew.-% und insbesondere 0,1 bis 10 Gew.-% bezogen auf das Gesamtgewicht der Zubereitung.

Geeignete kationische Polymere sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quaterniertes Chitosan, gegebenenfalls mikrokristallin verteilt. Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen sowie beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

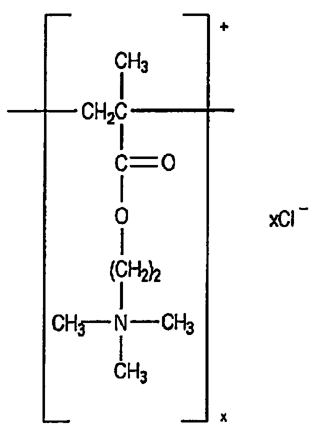

Ebenso geeignet sind sogenannte quaternäre Polymere, z.B. mit der INCI - Bezeichnung Polyquaternium-37, die der folgenden allgemeinen Formel entsprechen: Alternativ können auch andere Dialkylaminoalkyl (meth)acrylate sowie deren durch Alkylierung oder Protonierung erhältliche Ammoniumsalze oder Dialkylaminoalkyl (meth)acrylamide sowie deren durch Alkylierung oder Protonierung erhältliche Ammoniumsalze eingesetzt werden. Besonders bevorzugt sind Polymere enthaltend MAPTAC, APTAC, MADAME, ADAME, DMAEMA und TMAEMAC. Darüberhinaus können auch Copolymere mit anionischen, weiteren kationischen oder ungeladenen Monomeren erfindungsgemäß eingesetzt werden, insbesondere solche, die neben den genannten Alkylaminoalkyl (meth)acrylat oder -(meth)acrylamid Monomeren zusätzlich (Meth)acrylsäure und/oder 2-Acrylamido-2-methyl-propansulfonsäure und/oder Acrylamid und/oder Vinylpyrrolidon und/oder Alkyl(meth)acrylate enthalten.

Beispielhaft seien solche Polymere mit der INCI Bezeichnung Polyquaternium-11, Polyquaternium-13, Polyquaternium-14, Polyquaternium-15, Polyquaternium-28, Polyquaternium-32, Polyquaternium-43, Polyquaternium-47 genannt.

### Ölkörper

In einer Ausführungsform der Erfindung enthalten die erfindungsgemäßen Zubereitungen mindestens einen Ölkörper. In einer Ausführungsform der Erfindung enthalten die erfindungsgemäßen Zubereitungen die erfindungsgemäßen verzweigten Kohlenwasserstoffe als Ölkörper. In der hier als bevorzugte genannten Ausführungsform enthalten die Zubereitungen somit einen von dem erfindungsgemäßen verzweigten Kohlenwasserstoffen verschiedenen Ölkörper, auch als "weiterer Ölkörper" bezeichnet.

Ein Gegenstand der Erfindung betrifft kosmetische und/oder pharmazeutische Zubereitungen, enthaltend 0,1 bis 80 Gew.% der erfindungsgemäßen verzweigten Kohlenwasserstoffe und mindestens einen (weiteren) Ölkörper.

Die Ölkörper (erfindungsgemäße verzweigte Kohlenwasserstoffe plus weitere Ölkörper) sind üblicherweise in einer Gesamtmenge von 0,1 - 90, insbesondere 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% -bezogen auf das Gesamtgewicht der Zubereitung- enthalten.

Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen und Kohlenwasserstoffen oder deren Gemische.

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 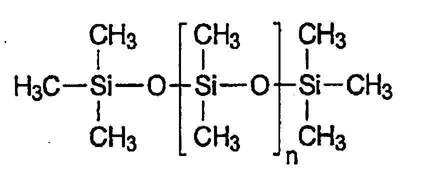 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handelsbezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclo- trisiloxan, Polydimethylsiloxan, Poly (methylphenylsiloxan). Erfindungsgemäß besonders bevorzugte Silikone sind Dimethicon und Cyclomethicon.

Die erfindungsgemäßen Zubereitungen können weiterhin biogenen Wirkstoffe, Insekten-Repellentien, Tyrosinase Inhibitoren, Konservierungsmittel, Parfümöle, Überfettungsmittel, Stabilitsatoren und/oder Hydrotrope enthalten.

Ein Gegenstand der Erfindung betrifft kosmetische und/oder pharmazeutische Zubereitungen, enthaltend 0,1 bis 80 Gew.% der erfindungsgemäßen verzweigten Kohlenwasserstoffe und mindestens einen biogenen Wirkstoff, Insekten-Repellent, Tyrosinase Inhibitor, Konservierungsmittel, Parfümöl, Stabilisator und/oder Hydrotrop.

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Aloe vera, Prunusextrakt, Bambaranussextrakt und Vitaminkomplexe zu verstehen.

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe. Weiterhin eignen sich als Konservierungsmittel die in WO07/048757 beschriebenen 1,2 Alkandiole mit 5 bis 8 C-Atomen.

Als Konservierungsmittel eignen sich insbesondere die gemäß Annex VI der Kommissions Direktive (in der Fassung Commission Directive 2007/22/EC of 17 April 2007 amending Council Directive 76/768/EEC**,** concerning cosmetic products, for the purposes of adapting Annexes IV and VI thereto to technical progress) zugelassen Stoffe, auf die hier explizit Bezug genommen wird.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als Stabilisatoren können Metallsalze von Fettsäuren, wie z. B. Magnesium-, Aluminium-und/oder Zinkstearat bzw. -ricinoleat eingesetzt werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Beispiele

### Herstellbeispiel 1: Herstellung erfindungsgemässer verzweigter Kohlenwasserstoffe aus einem primären Alkohol

Zur Herstellung der erfindungsgemäßen verzweigten Kohlenwasserstoffe wurde ein kontinuierlich betriebener 1-L- Reaktor mit einem Nickel-Katalysator (Johnson Matthey Pricat Ni 55/5 T) befüllt und der Katalysator entsprechend der Vorschrift des Herstellers aktiviert. Es wurde ein Wasserstoffdruck von 250 bar, ein Wasserstoffvolumenstrom von 15 Nm3/h und eine Reaktionstemperatur von 260°C eingestellt. Das Edukt Isostearylalkohol (Prisorine 3515 Isostearyl alcohol, Fa. Unicema, Gouda, Niederlande) wurde im Gleichstrom zum Gas mit 400 g/h in den Reaktor gegeben. Im Hydrierprodukt war kein Edukt (Isostearylalkohol) nachweisbar (Nachweisgrenze 0,01 %).

### Herstellbeispiel 2: Herstellung erfindungsgemässer verzweigter Kohlenwasserstoffe aus einem primären Dimerdiol

Zur Herstellung der erfindungsgemäßen verzweigten Kohlenwasserstoffe wurde ein kontinuierlich betriebener 1-L- Reaktor mit einem Nickel-Katalysator (Johnson Matthey Pricat Ni 55/5 T) befüllt und der Katalysator entsprechend der Vorschrift des Herstellers aktiviert. Es wurde ein Wasserstoffdruck von 250 bar, ein Wasserstoffvolumenstrom von 15 Nm3/h und eine Reaktionstemperatur von 260 °C eingestellt. Das Edukt Dimerdiol (Sovermol 908, Fa. Cognis GmbH, Düsseldorf) wurde im Gleichstrom zum Gas mit 400 g/h in den Reaktor gegeben. Im Hydrierprodukt betrug die Konzentration an verzweigten Kohlenwasserstoffen 35 %.

## Patentansprüche

1. Verzweigte Kohlenwasserstoffe, erhältlich durch Dehydroxymethylierung von Edukten ausgewählt aus der Gruppe bestehend aus
a) **verzweigten Alkoholen** der allgemeinen Formel R₁-OH, wobei R₁ für einen verzweigten Alkyl und/oder Alkenylrest mit 10 bis 29 C Atomen steht,
b) **verzweigten primären Diolen** der allgemeinen Formel HO-X-OH, wobei X für einen verzweigten Alkyl und/oder Alkenylrest mit 11 bis 44 C Atomen steht
c) **verzweigten primären Triolen** mit 12 bis 66 C Atomen.

2. Verzweigte Kohlenwasserstoffe nach Anspruch 1, **dadurch gekennzeichnet, dass** als verzweigte Alkohole R₁-OH **primäre** Alkohole eingesetzt werden, vorzugsweise Isostearylalkohol oder Isotridecylalkohol.

3. Verzweigte Kohlenwasserstoffe nach Anspruch 1, **dadurch gekennzeichnet, dass** als verzweigte primäre Diole **Dimerdiole** eingesetzt werden.

4. Verzweigte Kohlenwasserstoffe nach Anspruch 1, **dadurch gekennzeichnet, dass** als verzweigte primäre Triole **Trimertriole** eingesetzt werden.

5. Verzweigte Kohlenwasserstoffe nach einem der vorgenannten Ansprüche, **dadurch gekennzeichnet, dass** sie kleiner gleich 20 Gew.-%, insbesondere kleiner gleich 10 Gew.-%, insbesondere kleiner gleich 5 Gew.-%, bevorzugt kleiner gleich 1 Gew.-%, **ungesättigte verzweigte Kohlenwasserstoffe** bezogen auf die Summe der verzweigten Kohlenwasserstoffe enthalten.

6. Verzweigte Kohlenwasserstoffe nach einem der vorgenannten Ansprüche, **dadurch gekennzeichnet, dass** die Edukte einen Anteil der ¹⁴C Isotope zu den ¹²C Isotopen von größer gleich 1x10⁻¹⁶ aufweisen, vorzugsweise einen Anteil an ¹⁴C Isotopen zu ¹²C Isotopen im Bereich von 6 x 10⁻¹³ bis 1,2 x 10⁻¹² aufweisen.

7. Verzweigte Kohlenwasserstoffe nach einem der vorgenannten Ansprüche, **dadurch gekennzeichnet, dass** man die Dehydroxymethylierung bei Temperaturen von 180 bis 300°C, vorzugsweise 200 bis 280°C und insbesondere von 220 bis 260°C durchführt.

8. Verzweigte Kohlenwasserstoffe nach einem der vorgenannten Ansprüche, **dadurch gekennzeichnet, dass** man die Dehydroxymethylierung bei Drücken von 2 bis 300 bar, insbesondere von 2 bis 250 bar, insbesondere von 5 bis 100 bar, vorzugsweise von 5 bis 80 bar und insbesondere von 10 bis 50 bar durchführt.

9. Verzweigte Kohlenwasserstoffe nach einem der vorgenannten Ansprüche, **dadurch gekennzeichnet, dass** man die Dehydroxymethylierung in Gegenwart von Katalysatoren durchführt, insbesondere von Katalysatoren, die Nickel enthalten.

10. Verzweigte Kohlenwasserstoffe nach einem der vorgenannten Ansprüche, **dadurch gekennzeichnet, dass** man die Dehydroxymethylierung mit Katalysatorenmengen von 0,1 bis 3 Gew.-% vorzugsweise von 0,2 bis 2 Gew.-% und insbesondere von 0,5 bis 1,0 Gew.-%, jeweils bezogen auf die Menge an eingesetzten Edukten durchführt.

11. Verzweigte Kohlenwasserstoffe nach einem der vorgenannten Ansprüche, **dadurch gekennzeichnet, dass** man die Dehydroxymethylierung in Gegenwart von Wasserstoff, einem Katalysator und bei Temperaturen von 100 bis 300°C und Drücken von 2 bis 300 bar durchführt, wobei während der Reaktion entstehendes Wasser entfernt wird.

12. Verwendung von verzweigten Kohlenwasserstoffen nach einem der vorgenannten Ansprüche in kosmetischen und/oder pharmazeutischen Zubereitungen, insbesondere als Ölkörper und/oder als Wachskomponente.

13. Kosmetische und/oder pharmazeutische Zubereitungen, enthaltend 0,1 bis 80 Gew.% verzweigte Kohlenwasserstoffe nach einem der Ansprüche 1 bis 11.

14. Kosmetische und/oder pharmazeutische Zubereitung nach Anspruch 13, enthaltend mindestens eine Verbindung ausgewählt aus der Gruppe bestehend aus Antiperspirant/Desodorant Wirkstoffen, UV-Lichtschutzültem, Selbstbräunern, Pigmenten, Farbstoffen, Emulgatoren, Tensiden, Wachskomponenten, Polymeren und Ölkörpern.
